Europäisches Patentamt

(19) European Patent Office

Office européen des brevets

(11) Publication number : **0 420 713 B1**

(12) # EUROPEAN PATENT SPECIFICATION

(45) Date of publication of patent specification :
18.11.93 Bulletin 93/46

(51) Int. Cl.⁵ : **A61K 7/00, A61K 7/48**

(21) Application number : **90402499.9**

(22) Date of filing : **11.09.90**

(54) **Composition and method for coloring and moisturizing the skin.**

(30) Priority : **25.09.89 US 412018**

(43) Date of publication of application :
**03.04.91 Bulletin 91/14**

(45) Publication of the grant of the patent :
**18.11.93 Bulletin 93/46**

(84) Designated Contracting States :
**BE CH DE FR GB LI NL SE**

(56) References cited :
**EP-A- 0 138 635**
**GB-A- 2 074 584**
**US-A- 3 630 654**

(56) References cited :
**CHEMICAL ABSTRACTS, vol. 102, no. 24, 17th June 1985, page 344, abstract no. 209242z, Columbus, Ohio, US; B. HAIJRATWALA: "The stability of FD & C blue no. 1", & DRUG COSMET IND. 1985, 136(2), 50, 52, 86**
**Hawley's Condensed Chem. Dict., 11th Ed., Van Nostrand Reinhold Co., New York, USA, p. 682**

(73) Proprietor : **SHAGBARK RESEARCH, INC.**
**Barn Hill Road**
**Greenwich, Connecticut 06831 (US)**

(72) Inventor : **Menkart, John**
**Barn Hill Road**
**Greenwich, Connecticut 06831 (US)**
Inventor : **Hill, Ira D.**
**Clay Court**
**Locust, New Jersey 07760 (US)**

(74) Representative : **Rodhain, Claude et al**
**Cabinet Claude Rodhain 30, rue la Boétie**
**F-75008 Paris (FR)**

EP 0 420 713 B1

## Description

The present invention provides for the enhancement of natural facial color together with moisturization by using a single composition.

Various types of moisturizing skin care products are on the market. Moisturizers in the form of an emulsion are known, which may include, for example, water, glycerin, vitamin E, and lanolin, as well as additional ingredients. These products may further include small concentrations of fragrance and color, to enhance the commercial appeal of the product.

For instance, US-A-3 630 654 describes a composition in which an acid dye is contained in the oil phase of an emulsion, said oil phase consisting of a slightly water-soluble alcoholic hydroxy group containing solvent.

GB-A-2 074 584 makes use of salts of such acid dyes with copolymer carrying tertiary amine groups, for avoiding the "grip", namely the colouring of skin, mucous membrane or nail.

Another skin care formulation is described by Soma in US-A- 4 375 480. The disclosed moisturizing composition of Soma contains water, unsaturated vegetable oil, glycerin, triethanolamine, cetyl alcohol and selected emulsifiers. The Soma moisturizing composition may also contain small concentrations of water-soluble acid dyes employed to improve the appearance of the product by imparting color to the moisturizing formulation, as in traditional moisturizers.

Conventional moisturizers, such as the Soma formulation, are used in a daily make-up regimen to provide the function of moisturizing the skin. These moisturizers are typically used in conjunction with a traditional make-up formulation which provides the function of imparting color to the skin.

Two major methods currently exist for imparting color to the skin, each having its own disadvantages and deficiencies.

The most common approach, employed both in general-purpose women's make-up and in theatrical make-up, consists in the application of a waxy or oily coating, primarily colored with a mixture of finely-divided, solid, opaque pigments such as iron oxide and titanium dioxide. Cosmetic foundations, pancake make-ups, rouges and blushes are in this category.

This conventional coating acts in the manner of a paint, masking the substrate (i.e., the skin) and presenting its own surface structure and color to the observer. While this has the benefit of being able to cover blemishes, the result is an unnatural appearance and the clogging of pores, leading to all the known problems associated therewith.

Skin make-up products designed for simultaneously moisturizing and imparting color to mask the skin have been reported. For example, Calvo US-A-4 216 201 describes a water-in-oil lotion having skin moisturizing properties, which may be formulated as a carrier with cosmetic covering and coloring agents. The Calvo formulation functions to color the skin in a like manner as other traditional make-up formulations.

Negative aspects of these traditional make-up formulations, including those to which moisturizing ingredients have been added, include rub-off and the unsightly transfer of the coating to any surface coming in contact with the skin in addition to the above-mentioned problems of causing an unnatural appearance and clogging of the pores. Streaking on exposure to water or perspiration also tends to occur.

The second method, employed in products known as bronzers, utilizes solutions of acid dyes in an aqueous gel system to apply color which is partly absorbed into the skin and partly deposited on its surface. Consequently, the natural appearance of the skin is preserved (and blemishes are not masked), but the color is weakly attached to the skin, so that some of it is readily removed by rubbing and much of it is lost by simple rinsing with water or by perspiration.

An improvement in the bronzer system has recently been made by L. Albrecht and L. J. Wolfram, consisting in the coloring of skin with a formulation containing an aromatic or aliphatic alcohol, or a mixture thereof, in an aqueous base, the composition having an acidic pH. This improved bronzer system is described in U.S. Serial No. 231,732, filed August 12, 1988 now US-A- 5 015 263, issued on May 14, 1991, the disclosure of which is herein incorporated by reference. The Albrecht and Wolfram composition enhances the uptake of the dyes by the skin, and greatly increases the resistance to water and perspiration, though the color can be readily removed by washing with soap.

The Albrecht and Wolfram system, like other bronzers, is rather messy to apply. Another disadvantage is that a good deal of skill is required to perform the application rapidly and smoothly, to avoid streaks or sharp edges caused by the enhanced and rapid uptake of the dyes by the skin. A further drawback is that the application of the bronzer represents an additional, time-consuming step in the make-up regimen.

An additional deficiency is that the bronzing gel product described by Albrecht and Wolfram is somewhat drying to the skin. If a moisturizer, which is routinely employed by most women in their daily make-up regimen, is used along with the gel product, the effectiveness of the bronzer system is detrimentally diminished. When a moisturizer is separately applied prior to the gel, the resistance of the color to subsequent rubbing and water-rinsing is impaired. When the moisturizer is applied after the gel, the intensity of the color is greatly decreased as the moisturizer is worked into the skin, and the color tone tends to change. Such deleterious effects are shown below in Table I.

## TABLE I

| PROCESS | COLOR INTENSITY | | |
|---|---|---|---|
| | Initial | Rinsed | Soaped |
| **Pretreatment** | | | |
| None | 100 | 90 | 20 |
| (1) | 100 | 70 | 10 |
| (2) | 100 | 70 | 10 |
| (3) | 100 | 60 | 0 |
| **Posttreatment** | | | |
| None | 100 | 90 | 20 |
| (1) | 20 | 10 | 5 |
| (2) | 25 | 20 | 10 |
| (3) | 10 | 5 | 0 |

(1)  Vaseline Intensive Care Lotion® Extra Strength
(2)  Rite Aid Skin Care Lotion®
(3)  Avon VitaMoist Body Lotion®

An object of the present invention is to overcome the above-mentioned disadvantages and deficiencies of the known methods for imparting color into the skin.

Another object of the invention is to provide a single bronzer-type composition for moisturizing and imparting color into the skin.

The present inventors have surprisingly discovered that by combining the skin coloring system described by Albrecht and Wolfram with the ingredients of an effective facial moisturizer, a novel, advantageous and more useful product in the form of a lotion or cream is obtained, which is easy to apply.

The product according to this invention spreads on the skin much more easily, and the color is absorbed by the skin more slowly. Consequently, a satisfactory result is readily realized without expertise or detailed instruction in the technique of applying the product.

An additional advantage of the invention is that after the product is absorbed into the skin, the durability (i.e., the resistance to rubbing, water and perspiration) is surprisingly equal to that delivered by the gel of Albrecht and Wolfram (see the product performance data set forth in Table V). This result is in contrast with the typical reduction in dye binding to the skin in the usual sequential treatment of the skin with a moisturizer and a dye -- i.e., both pre- and post-application of moisturizer diminish the performance of the color. See Table I above.

Other valuable characteristics of the inventive product are its unusually pleasant, non-oily feel and its ready spreadability without the foaming often resulting from vigorously working an oil-in-water emulsion.

These desirable properties are achieved by the present invention, which utilizes a dual thickening system. A dual thickening system useful in the invention employs a gellant for the aqueous phase and surfactant emulsifying agents to create the composition.

Compositions according to this invention have a variety of uses. They may be utilized to produce enhancement of natural facial color. For example, a person who is tanned in the summer can use a lightly colored formulation in other seasons to eliminate "winter pallor". For this purpose, the composition can be used alone, or conventional make-up can be applied on top of it. More heavily colored formulations may be employed to simulate a suntan, without exposing the skin to the harmful effects of ultraviolet radiation -- an inevitable concomitant of exposure to sunlight. Furthermore, a sunscreen may be incorporated in the formulation to minimize skin damage caused by sun exposure.

Another important application of the product is in the cosmetic treatment of skin depigmentation, such as occurs in the case of the disease vitiligo or in surgical or burn scars. For such uses, typical methods of application which permit controlled delivery to exactly delineated portions of the skin are used.

For the uses outlined above, compositions matching or enhancing natural skin color are employed. The product also can be formulated to produce "fantasy shades or colors" (i.e., colors outside the range of natural skin tones) that are of value for theatrical purposes and for special applications, such as children's Halloween make-up.

The composition according to the present invention comprises :

(a) an aqueous phase containing

(i) an acid dye component at a concentration effective to impart color to the skin comprising at least one water-soluble acid dye possessing at least one sulfonic group, and

(ii) an alcohol component selected from the group consisting of aromatic alcohols, aliphatic alcohols and mixtures thereof ; and,

(b) an oil phase dispersed in said aqueous phase, said oil phase comprising an emollient and at least one emulsifying agent,

wherein the composition has a pH equal to or less than 7.

The dyes useful for this invention are water-soluble acid dyes possessing at least one sulfonic group. For a product to be applied to human skin, the selection of dyes is restricted to those listed by the Food and Drug Administration as FD&C, D&C or Ext.D&C dyes.

Preferred dyes are (Color Index designation in parentheses) FD&C Blue 1 (Acid Blue 9), FD&C Red 4 (Ponceau SX), FD&C Yellow 5 (Acid Yellow 23), FD&C Yellow 6 (Sunset Yellow), D&C Green 5 (Acid Green 25), D&C Orange 4 (Acid Orange 7), D&C Yellow 10 (Acid Yellow 3), Ext.D&C Violet 2 (Acid Violet 43), and Ext.D&C Yellow 7 (Acid Yellow 1). A preferred range of concentrations of the dye component is from about 0.02 to about 1.0 % by weight, and more preferably from about 0. 05 to about 0.50 % by weight.

Conventional polymeric thickeners or gelling agents are used to thicken the aqueous phase of the formulation. Examples are carboxymethyl cellulose, hydroxyethyl cellulose and guar gum. Particularly preferred as a thickening agent is polyacrylic acid (CTFA designation Carbomer 940 or Carbomer 934P), which leaves little residual tacky feel on the skin.

The uptake of the dye by the skin and the tenacity with which it is held in the skin, depend largely on the moiety and concentration of alcohol present in the composition and the pH of the composition. Alcohols useful in this invention are aliphatic and aromatic compounds capable of penetrating the skin and altering its hydrophobic or hydrophilic nature, are liquid at room temperature, possess at least some water solubility, and have no objectionable odor, irritation or toxicity under use conditions. Preferred alcohols are benzyl alcohol, n-pentanol, n-butanol, ethanol and mixtures thereof. A particularly preferred alcohol component is made of a mixture of benzyl alcohol and ethanol. The preferred concentration of alcohol in the composition is from about 1 % to about 40 % by weight.

The pH of the composition should be less than 7, and preferably is below about 5.5. At pH values less than 3, the rate of uptake of the dye by the skin ("strike") becomes so rapid than an even, non-streaky effect is difficult to attain. The pH is adjusted to the chosen value by means of an organic base, such as triethanolamine or morpholine, or by means of an acid such as citric acid if lowering is necessary.

The moisturizing effect is incorporated in the product by way of lipids such as lanolin, triglycerides of organic acids with a chain length of eight or more, mineral oil and other conventional emollients, used alone or in mixtures. If the product incorporates a sunscreen, and if the substance employed for this purpose is an oil such as ethylhexyl methoxycinnamate, this substance can fill the role of part or all of the emollient component. The emollient can represent between 0.5 and 10 % by weight of the total composition.

Emulsification is accomplished by means of a mixture of hydrophilic and hydrophobic emulsifying agents or surfactants familiar in the art for the formation of oil-in-water emulsions. A mixture of sodium cetearyl sulfate, ceteareth 20, cetearyl alcohol and self-emulsifying glyceryl stearate is a useful emulsifying agent.

Other ingredients may be incorporated in the composition for specific purposes. For example, it is usually necessary to include antimicrobial agents to protect the formulation from being degraded by bacterial or fungal attack. A fragrance may be added to mask undesirable odors or to confer a desirable scent. A humectant, such as glycerin or propylene glycol, can be included to slow the drying-out of the product when a container is left open, as well as to enhance the perceived moisturization of the skin.

The formulation may also contain a water-repelling agent, such as dimethicone. The presence of a water repellent can further increase the water- and perspiration resistance of the skin coloration, and of the sunscreen when the latter is present, as well as add lubricity.

In order to secure a natural and pleasing appearance, the tone of color imparted by the product must complement the skin color of the subject, although it is not necessary that the tone of color imparted exactly match the skin color. Table II below lists five dye compositions which, when employed in the formulations given in Tables III and IV as follow, deliver a natural appearance with the tonality indicated. The terminology used to name the ingredients in the Tables is that of the <u>CTFA Cosmetic Ingredients Dictionary.</u>

TABLE II

| Dye | Tone | Pink | Rose | Peach | Olive | Tropic |
|-----|------|------|------|-------|-------|--------|
| FD&C Blue 1 | | .0071 | .0093 | .0116 | .0115 | 0.0000 |
| FD&C Red 4 | | .0761 | .0429 | .0429 | .0098 | 0.0000 |
| FD&C Yellow 6 | | 0.0000 | .0056 | .0056 | .0111 | 0.0000 |
| D&C Green 5 | | 0.0000 | 0.0000 | 0.0000 | 0.0000 | .0522 |
| D&C Orange 4 | | 0.0000 | .0364 | .0364 | .0728 | .0752 |
| Ext.D&C Violet 2 | | .0027 | .0142 | .0128 | .0215 | .0365 |
| Ext.D&C Yellow 7 | | .0217 | .0103 | .0114 | 0.0000 | 0.0000 |
| Total | | .1076 | .1187 | .1207 | .1267 | .1639 |

## TABLE III

| INGREDIENT | % BY WEIGHT |
|---|---|
| EMOLLIENT [1] | 0.50 -10.00 |
| Glyceryl Stearate | 0.40 - 3.00 |
| Sodium Cetearyl Sulfate | 0.40 - 3.00 |
| Cetearyl Alcohol | 0.20 - 1.60 |
| Ceteareth 20 | 0.00 - 1.60 |
| Dimethicone | 0.50 - 3.00 |
| Glycerin | 0.50 - 3.00 |
| Benzyl Alcohol | 0.50 - 4.00 |
| SD Alcohol 40 | 0.00 - 6.00 |
| Carbomer 940 | 0.15 - 0.60 |
| Methylparaben | 0.15 |
| Propylparaben | 0.05 |
| DMDM Hydantoin | 0.10 |
| Dyes (Table II) | 0.02 - 1.00 |
| Triethanolamine | q.s. to pH desired |
| Water | q.s. 100.00 |

[1] EMOLLIENT selected form one or more of:
    Capryllic/Capric Triglyceride
    Mineral Oil
    Petrolatum
    Stearyl Stearate
    Vegetable Oil
    Lanolin
    Stearic Triglyceride (Cetiol R, Henkel)
    Longchain Hydrocarbon (Cetiol S, Henkel)

6

## TABLE IV

| INGREDIENT | % BY WEIGHT | | |
|---|---|---|---|
| | A | B | C |
| Capryllic/Capric Triglyceride | 3.00 | .50 | -- |
| Ethylhexyl Methoxycinnamate | -- | 3.00 | 5.00 |
| Glyceryl Stearate | 1.25 | 1.50 | 1.75 |
| Sodium Cetearyl Sulfate | 1.25 | 1.25 | 1.75 |
| Cetearyl Alcohol | .80 | .80 | 1.00 |
| Ceteareth 20 | .70 | .70 | .87 |
| Dimethicone | 1.00 | 1.00 | 1.00 |
| Glycerin | 1.00 | 1.00 | 1.00 |
| Benzyl Alcohol | 2.00 | 2.00 | 2.50 |
| SD Alcohol 40 | .75 | .75 | 1.00 |
| Carbomer 940 | .45 | .40 | .35 |
| Methylparaben | .15 | .15 | .15 |
| Propylparaben | .05 | .05 | .05 |
| DMDM Hydantoin | .10 | .10 | .10 |
| Dyes | 05-.50 | .05-.50 | .05-.50 |
| Triethanolamine | (about) .18* | (about).18* | (about).18* |
| Water | q.s.100.00 | q.s.100.00 | q.s.100.00 |

\* Quantity required to adjust pH to 4.7-4.9

In Table III, a range of ingredients and concentrations are presented by which efficacious formulations of the present invention may be prepared. Table IV describes three variants of a particularly effective and pleasant formulation, these products being best described as a "cream" and having a viscosity in the range of 600-900 Pa.s (600,000-900,000 cP). Two of the variants, B and C, contain a sunscreen. Variant B has a Sun Protection Factor (SPF) of about 4, and C has an SPF of about 6.

These and similar formulations can be readily compounded and packaged by conventional procedures with conventional equipment for the manufacture of cosmetic creams and lotions.

Comparative Product Performance Data

The data of Table V below compare the rate of color uptake by the skin and the resistance to water rinsing for the product of this invention ("cream") and the gel of Albrecht and Wolfram ("gel"). Formulations having the same shade (i.e., the Rose shade of Table II) were employed. To generate these data, each product was applied to small spots on the hairless inner forearm, and the excess was wiped off after the specified time. The depth of color was then estimated visually on a scale of 0-100, the arm was rinsed for 30 seconds under warm running water, dried and the residual color was evaluate.

TABLE V

| Application Time (Seconds) | Color Intensity | | | |
|---|---|---|---|---|
| | Cream | | Gel | |
| | Initial | Rinsed | Initial | Rinsed |
| 20 | 20 | 10 | 70 | 40 |
| 40 | 40 | 25 | 90 | 80 |
| 80 | 70 | 50 | 100 | 90 |
| 160 | 100 | 90 | 100 | 90 |

From the above results, it is evident that the skin is colored much more slowly by the cream; yet, when the process is complete, the resistance to removal by water rinsing is equal to that delivered by the gel system.

Another experiment was performed in a similar manner to demonstrate the effect of pre- and post-application of several commercial moisturizers on the uptake of color from the gel by the skin, and on its subsequent removal by water-rinsing, followed by a light wash with soap. In this test, the gel was allowed to absorb for 80 seconds before wiping off the excess. The results, which have been summarized in the foregoing in Table I, show that pre-treatment of the skin with a moisturizer diminishes the resistance of the color to rinsing, and that post-application of moisturizer dramatically weakens the color as well as changes the tonality, in this instance in the blue direction.

## Claims

1. A composition for coloring and moisturizing skin, comprising:
   (a) an aqueous phase containing
   (i) an acid dye component at a concentration effective to impart color to the skin comprising at least one water-soluble acid dye possessing at least one sulfonic group, and
   (ii) an alcohol component selected from the group consisting of aromatic alcohols, aliphatic alcohols and mixtures thereof;
   and,
   (b) an oil phase dispersed in said aqueous phase, said oil phase comprising an emollient and at least one emulsifying agent,
   wherein the composition has a pH equal to or less than 7.

2. A composition according to claim 1, wherein the alcohol component is selected from the group consisting of benzyl alcohol, n-pentanol, n-butanol, ethanol and mixtures thereof.

3. A composition according to claim 1, wherein the alcohol component consists of a mixture of benzyl alcohol and ethanol.

4. A composition according to claim 1, wherein the pH of the composition is between 3.0 and 5.5.

5. A composition according to claim 1, wherein the composition contains a sunscreen.

6. A composition according to claim 5, wherein said sunscreen is ethylhexyl methoxycinnamate.

7. A composition according to claim 1, wherein the composition contains from 0.02 to 1.0 percent by weight of said acid dye component.

8. A composition according to claim 1, wherein the composition contains from 1 to 40 percent by weight of said alcohol component.

9. A composition according to claim 1, wherein said emollient is selected from the group consisting of lipids, triglycerides of organic acids having a chain length of at least eight, mineral oils, and mixtures thereof.

10. A composition according to claim 1, wherein said emollient is a lipid.

11. A composition according to claim 1, further comprising a water repellent.

12. A composition according to claim 1, wherein said aqueous phase further contains a thickening agent.

13. A composition according to claim 1, wherein the acid dye component is effective to impart a color tone which enhances natural skin color.

14. A composition according to claim 1, wherein the acid dye component is effective to impart a natural skin color to decolorized portions of the skin.

15. A composition according to claim 1, wherein the acid dye component is effective to impart a fantasy color to the skin.

16. A process for moisturizing and imparting color to skin, comprising contacting the skin with a composition comprising:
    (a) an aqueous phase containing
        (i) an acid dye component at a concentration effective to impart color to the skin comprising at least one water-soluble acid dye possessing at least one sulfonic group, and
        (ii) an alcohol component selected from the group consisting of aromatic alcohols, aliphatic alcohols and mixtures thereof;
            and,
    (b) an oil phase dispersed in said aqueous phase, said oil phase comprising an emollient and at least one emulsifying agent,
    wherein the composition has a pH equal to or less than 7.

17. A process according to claim 16, wherein said emollient is a lipid and the alcohol component is selected from the group consisting of benzyl alcohol, n-pentanol, n-butanol, ethanol and mixtures thereof.

18. A process according to claim 16, wherein the acid dye component is effective to impart a color tone which enhances natural skin color.

19. A process according to claim 16, wherein the acid dye component is effective to impart a natural skin color to decolorized portions of the skin.

20. A process according to claim 16, wherein the acid dye component is effective to impart a fantasy color to the skin.


**Patentansprüche**

1. Mittel zum Färben und zur Feuchthaltung der Haut bestehend aus:
    a) einer wässrigen Phase mit
        I) einer sauren Farbkomponente, in einer Konzentration, die geeignet ist Farbe auf die Haut zu Übertragen und die aus wenigstens einem wasserlöslichen, sauren Farbstoff besteht, der mindestens eine Sulfo-Gruppe enthält, und
        II) einer Alkohol-Komponente, die aus einer aus aromatischen Alkoholen, aliphatischen Alkoholen und deren Mischungen bestehenden Gruppe ausgewählt ist; und
    b) einer in der wässrigen Phase dispergierten Öl-Phase, die einen Weichmacher und wenigstens einen Emulgator enthält und
    wobei das Mittel einen pH-Wert von kleiner oder gleich 7 aufweist.

2. Mittel nach Anspruch 1, bei dem die Alkohol-Komponente aus einer, aus Benzyl-Alkohol, n-Pentanol, n-Butanol, Ethanol und deren Mischungen bestehenden Gruppe ausgewählt ist.

3. Mittel nach Anspruch 1, bei dem die Alkohol-Komponente aus einer Mischung aus Benzyl-Alkohol und Ethanol besteht.

4. Mittel nach Anspruch 1, bei dem der pH-Wert des Mittels zwischen 3,0 und 5,5 beträgt.

5. Mittel nach Anspruch 1, bei dem das Mittel ein Sonnenschutzmittel enthält.

6. Mittel nach Anspruch 5, bei dem das Sonnenschutzmittel Ethyl-hexyl-Zimtsäuremethoxyester ist.

7. Mittel nach Anspruch 1, bei dem das Mittel zwischen 0,02 und 1 Gew.-% der sauren Farbkomponente enthält.

8. Mittel nach Anspruch 1, bei dem das Mittel zwischen 1 und 40 Gew.-% der Alkohol-Komponente enthält.

9. Mittel nach Anspruch 1, bei dem der Weichmacher aus einer, aus Lipiden, Triglyceriden organischer Säuren mit einer Kettenlänge von wenigstens 8, Mineralölen und deren Mischung bestehenden Gruppe ausgewählt ist.

10. Mittel nach Anspruch 1, bei dem der Weichmacher ein Lipid ist.

11. Mittel nach Anspruch 1 mit einem zusätzlichen Wasserabweiser.

12. Mittel nach Anspruch 1, bei dem die wässrige Phase zusätzlich ein Eindickungsmittel enthält.

13. Mittel nach Anspruch 1, bei dem die saure Farbkomponente geeignet ist, einen Farbton zu übertragen, der die natürliche Hautfarbe verstärkt.

14. Mittel nach Anspruch 1, bei dem die saure Farbkomponente geeignet ist, eine natürliche Hautfarbe auf bleiche Hautbereiche zu übertragen.

15. Mittel nach Anspruch 1, bei dem die saure Farbkomponente geeignet ist, eine Phantasiefarbe auf die Haut zu übertragen.

16. Verfahren zur Feuchthaltung und zum Auftragen von Farbe auf die Haut, bei dem auf die Haut ein Mittel aufgetragen wird, das besteht aus:
   a) einer wässrigen Phase mit
      I) einer sauren Farbkomponente, in einer Konzentration, die geeignet ist, Farbe auf die Haut zu übertragen und die aus wenigstens einem wasserlöslichen, sauren Farbstoff besteht, der mindestens eine Sulfo-Gruppe enthält, und
      II) einer Alkohol-Komponente, die aus einer, aus aromatischen Alkoholen, aliphatischen Alkoholen und deren Mischungen gebildeten Gruppe ausgewählt ist; und
   b) einer in der wässrigen Phase dispergierten Öl-Phase, die einen Weichmacher und wenigstens einen Emulgator enthält und wobei das Mittel einen pH-Wert von kleiner oder gleich 7 aufweist.

17. Verfahren nach Anspruch 16, bei welchem der Weichmacher ein Lipid ist und die Alkohol-Komponente aus einer, aus Benzyl-Alkohol, n-Pentanol, n-Butanol, Ethanol und deren Mischungen bestehenden Gruppe ausgewählt ist.

18. Verfahren nach Anspruch 16, bei welchem die saure Farbkomponente geeignet ist, einen Farbton auf die Haut zu übertragen, der die natürliche Hautfarbe verstärkt.

19. Verfahren nach Anspruch 16, bei welchem die saure Farbkomponente geeignet ist, eine natürliche Hautfarbe auf bleiche Hautbereiche zu übertragen.

20. Verfahren nach Anspruch 16, bei welchem die saure Farbkomponente geeignet ist, eine Phantasie-Farbe auf die Haut zu übertragen.

**Revendications**

1. Composition pour la coloration et l'hydratation de la peau, comprenant:

a) une phase aqueuse contenant

(I) un composant colorant acide à une concentration efficace pour conférer une couleur à la peau, comprenant au moins un colorant acide hydrosoluble comportant au moins un groupe sulfonique, et

(II) un composant alcool choisi parmi des alcools aromatiques, des alcools aliphatiques et des mélanges de ceux-ci;

et

b) une phase huileuse dispersée dans ladite phase aqueuse, ladite phase huileuse comprenant un émollient et au moins un agent émulsifiant, la composition ayant un pH égal ou inférieur à 7.

2. Composition selon la revendication 1, dans laquelle le composant alcool est choisi parmi l'alcool benzylique, le n-pentanol, le n-butanol, l'éthanol et des mélanges de ceux-ci.

3. Composition selon la revendication 1, dans laquelle le composant alcool consiste en un mélange d'alcool benzylique et d'éthanol.

4. Composition selon la revendication 1, dans laquelle le pH de la composition est compris entre 3,0 et 5,5.

5. Composition selon la revendication 1, caractérisée en ce que la composition contient un écran solaire.

6. Composition selon la revendication 5, dans laquelle l'écran solaire est le méthoxycinnamate d'éthylhexyle.

7. Composition selon la revendication 1, caractérisée en ce que la composition contient de 0,02 à 1,0 % en poids dudit composant colorant acide.

8. Composition selon la revendication 1, caractérisée en ce que la composition contient de 1 à 40 % en poids dudit composant alcool.

9. Composition selon la revendication 1, dans laquelle ledit émollient est choisi parmi des lipides, des triglycérides d'acides organiques ayant une longueur de chaine d'au moins 8, des huiles minérales et des mélanges de ceux-ci.

10. Composition selon la revendication 1, dans laquelle ledit émollient est un lipide.

11. Composition selon la revendication 1, comprenant en outre un agent hydrofuge.

12. Composition selon la revendication 1, dans laquelle la phase aqueuse contient en outre un épaississant.

13. Composition selon la revendication 1, dans laquelle le composant colorant acide est efficace pour conférer une nuance colorée qui accentue la couleur naturelle de la peau.

14. Composition selon la revendication 1, dans laquelle le composant colorant acide est efficace pour conférer une couleur de peau naturelle à des parties décolorées de la peau.

15. Composition selon la revendication 1, dans laquelle le composant colorant acide est efficace pour conférer une couleur artificielle à la peau.

16. Procédé pour hydrater la peau et lui conférer une coloration, comprenant la mise en contact de la peau avec une composition comprenant:

a) une phase aqueuse contenant

(I) un composant colorant acide à une concentration efficace pour conférer une couleur à la peau, comprenant au moins un colorant acide hydrosoluble comportant au moins un groupe sulfonique, et

(II) un composant alcool choisi parmi des alcools aromatiques, des alcools aliphatiques et des mélanges de ceux-ci;

et

(b) une phase huileuse dispersée dans ladite phase aqueuse, ladite phase huileuse comprenant un émollient et au moins un agent émulsifiant, la composition ayant un pH égal ou inférieur à 7.

17. Procédé selon la revendication 16, dans lequel ledit émollient est un lipide et le composant alcool est choisi parmi l'alcool benzylique, le n-pentanol, le n-butanol, l'éthanol et des mélanges de ceux-ci;

18. Procédé selon la revendication 16, dans lequel le composant colorant acide est efficace pour conférer une nuance colorée qui accentue la couleur naturelle de la peau.

19. Procédé selon la revendication 16, dans lequel le composant colorant acide est efficace pour conférer une couleur de peau naturelle à des parties décolorées de la peau.

20. Procédé selon la revendication 16, dans lequel le composant colorant acide est efficace pour conférer une couleur artificielle à la peau.